# EUROPEAN PATENT SPECIFICATION

(11) **EP 2 132 300 B1**
(45) Date of publication and mention of the grant of the patent: **12.01.2011**
(21) Application number: 08743728.1
(22) Date of filing: 07.03.2008
(51) Int. Cl.: C12N 5/09, C12N 5/071

(54) **CELLS FOR ADENOVIRUS VECTOR AND PROTEIN PRODUCTION**
ZELLEN FÜR ADENOVIRUSVEKTOR- UND PROTEINHERSTELLUNG
CELLULES POUR LA PRODUCTION DE PROTÉINES ET DE VECTEURS ADÉNOVIRAUX

(30) Priority: 09.03.2007 US 894018 P
(43) Date of publication of application: 16.12.2009
(73) Proprietor: Vectorlogics, Inc., Birmingham, AL 35222 (US)
(72) Inventor: KOVESDI, Imre, Rockville, MD 20855-1033 (US)
(74) Representative: Svingor, Adam
(86) International application number: PCT/US2008/056198
(87) International publication number: WO 2008/112540

(56) References cited:
- EP-A- 0 905 253
- WO-A-00/72887
- WO-A-97/00326
- WO-A-99/57296
- US-A1- 2002 127 582
- US-B1- 6 541 245
- MCGRORY ET AL: "A SIMPLE TECHNIQUE FOR THE RESCUE OF EARLY REGION 1 MUTATIONS INTO INFECTIOUS HUMAN ADENOVIRUS TYPE 5" VIROLOGY, ACADEMIC PRESS,ORLANDO, US, no. 163, 1 January 1988 (1988-01-01), pages 614-617, XP002076238 ISSN: 0042-6822
- PEREZ C ET AL: "FACTORS AFFECTING DOUBLE-STRAND BREAK-INDUCED HOMOLOGOUS RECOMBINATION IN MAMMALIAN CELLS" BIOTECHNIQUES, INFORMA LIFE SCIENCES PUBLISHING, WESTBOROUGH, MA, US, vol. 39, no. 1, 1 July 2005 (2005-07-01), pages 109-115, XP008065842 ISSN: 0736-6205

## Description

### INCORPORATION BY REFERENCE

This application claims priority to U.S. provisional patent application Serial No. 60/894,018 filed March 9, 2007.

### FIELD OF THE INVENTION

The present invention relates to a novel cell line for adenovirus (Ad) and protein production that does not eliminate the overlap between the cell-line and adenovirus-derived vector sequences, but represses the frequency of undesirable homologous recombination events by the use of a large non-homologous spacer element(s).

### BACKGROUND OF THE INVENTION

Adenovirus vectors have attracted considerable interest over the past decade, with ongoing clinical development programs for applications ranging from replacement therapy for protein deficiencies to cancer therapeutics to prophylactic vaccines (reviewed by Altaras et al., Adv Biochem Eng Biotechnol. 2005;99:193-260). Consequently, considerable product, process, analytical, and formulation development has been undertaken to support these programs. For example, "gutless" vectors have been developed in order to improve gene transfer capacity and durability of expression, new cell lines have been developed to minimize undesirable recombination events, production conditions have been optimized to improve volumetric productivities, analytical techniques and scaleable purification processes have advanced towards the goal of purified adenovirus becoming a "well-characterized biological", and liquid formulations have been developed which maintain virus infectivity at 2-8 degrees C for over 18 months.

Cell lines for adenovirus ("Ad") production include such cell lines as human embryonic kidney ("HEK") 293 cells, 293-ORF6 cells and human embryonic retinoblasts ("HER") PER.C6 cells. A problem with these cell lines is either homologous recombination leading to the generation of undesirable replication competent adenovirus (RCA), such as is found with HEK 293 cells, or the incorporation of specific Ad genes that may impact on virus productivity or limit the type of vector whose replication it supports.

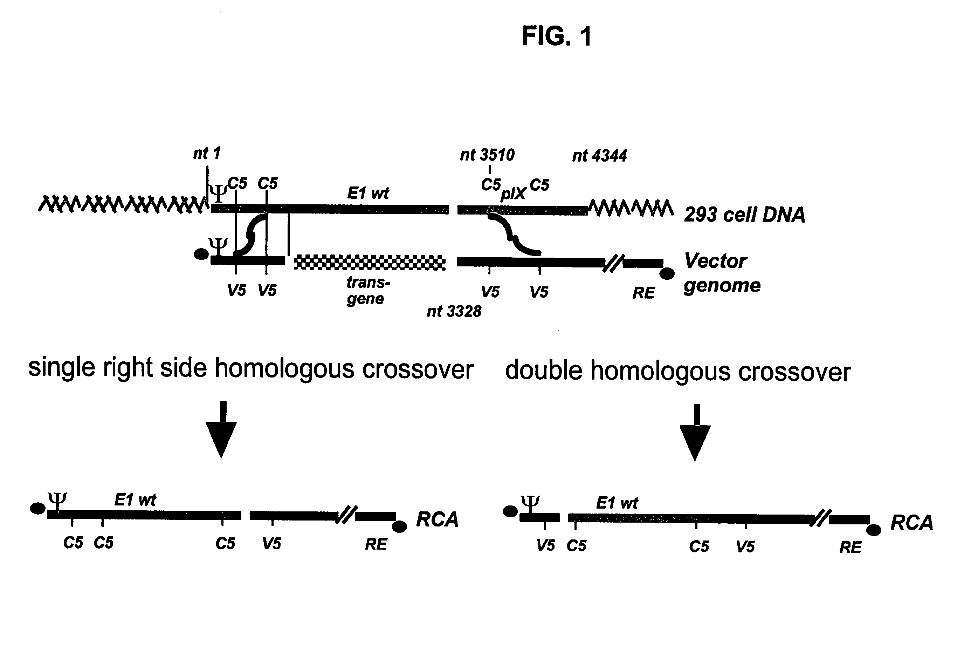

Most researchers use the HEK 293 cell-line when replication-deficient vectors are produced. It incorporates Ad sequences 1- 4344 nucleotides (nt) consisting of the Ad inverted terminal repeat ("ITR"), early region 1A ("E1A"), early region 1B ("E1B") and protein IX ("pIX") genes. E1A and E1B are essential regulatory genes, pIX codes for a non-essential capsid protein. This cell-line is able to complement Ad vectors deficient in E1A and E1B. Although pIX is expressed at a low level, it is not sufficient to complement for pIX deficiency. The most serious drawback of this cell-line is the production of replication competent adenovirus ("RCA") through homologous recombination between the overlapping Ad sequences in the cells and the vector. RCA is a safety issue for the FDA and other regulatory authorities. See, e.g., FIG. 1.

The cell line 293-ORF6 is based on 293 cells. This cell-line which also incorporates the E4-ORF6 Ad gene and has been specifically engineered to support the production of Ad vectors deficient in the E1A, E1B and E4 genes. Although, E1A and E1B deleted vectors can grow in this cell-line, only E1A, E1B and E4 deleted vectors can be grown without the occurrence of RCA. See, e.g., U.S. Patent Nos. 6,974,695, 6,913,922, 6,869,794, 6,579,522, 6,492,169 and 6,291,214.

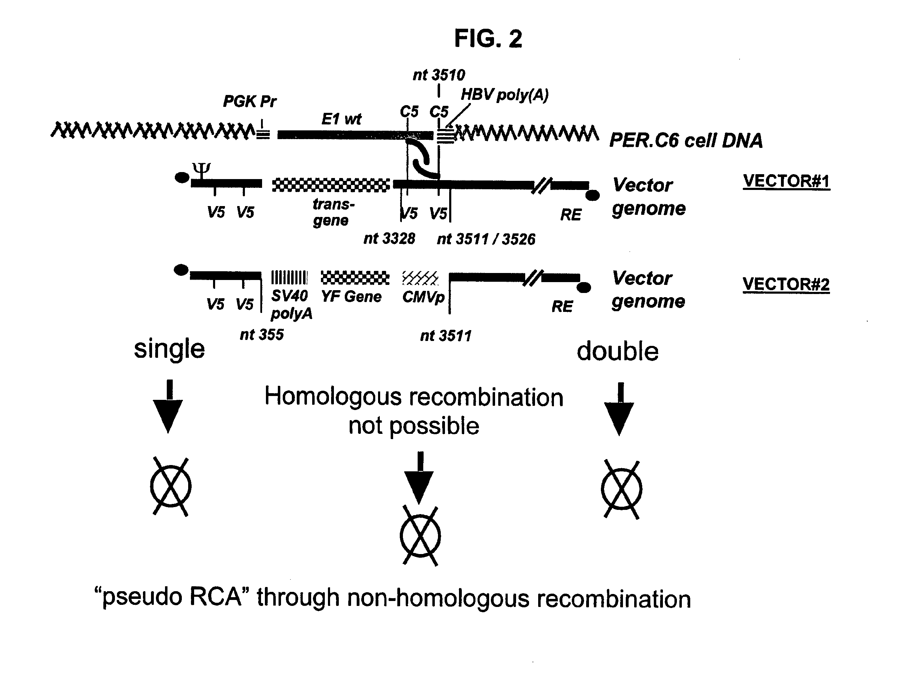

PER.C6 cells are based on primary retinoid cells. This cell-line incorporates Ad sequences 459- 3510 nt, comprising the Ad E1A and E1B genes, but not the pIX gene or the Ad ITR. The important aspect of this cell-line that E1A and E1B deleted Ad vectors may be produced without the production of RCA generated via homologous recombination since the overlap between the producer cells and many vector constructs has been eliminated. See, e.g., U.S. Patent Nos. 5,994,128, 6,265,212, 6,492,169, 6,602,706, 6,670,188, 6,692,966, 6,783,980, 6,855,544, 6,869,794 and 6,974,695 and FIG. 2. Ad vector yields from this cell line are typically reduced relative to their yield from HEK 293.

There remains a need for cell lines for Ad production that avoid the production of replication competent adenovirus while maintaining high vector yields.

Citation or identification of any document in this application is not an admission that such a document is available as prior art to the present invention.

### SUMMARY OF THE INVENTION

The present invention is based, in part, upon a novel cell-line based on 293 cells which represses undesirable homologous recombination events or their effects by the use of a large non-homologous spacer element(s).

The present invention relates to a mammalian packaging or production cell which may comprise a non-homologous spacer element inserted into an adenovirus nucleotide sequence, wherein the spacer element represses undesirable homologous recombination to reduce the frequency of production of replication-competent adenovirus when adenovirus vectors are propagated in the cell. Advantageously, the cell is a human embryonic kidney cell or 293 cell which can complement grows of adenovirus vectors deficient in the E1A and E1B genes. The spacer element may be inserted by site directed integration at a specific site in the adenovirus nucleotide sequence present in the packaging cell. Further spacer(s) might also be inserted into the already integrated spacer element by the use of specific site specific recombination element(s) such as, but not limited to, the lox, frt and attB sites used by the CRE, FLPe and phiC31 systems, respectively. Insertion of spacer element(s) can be also accomplished by the use of transposable element(s) as such as, but not limited to, Sleeping Beauty (SB) transposase. The specific site in the adenovirus nucleotide sequence may be after the end of the early region 1B ("E1B") transcription unit but in front of the protein IX ("pIX") transcription unit, after the inverted terminal repeat ("ITR") and packaging sequences but before the early region 1A ("E1A") transcription start site or after the E1A sequences but before the E1B sequences, or in other regions that do not interfere with their function.

There are several embodiments of the spacer element. In a first embodiment, the spacer element may comprise one or more regulatory elements such as, but not limited to, promoters, enhancers, insulators, polyadenylation and termination signals. In a second embodiment, the spacer element may be of varying sizes, such as, but not limited to, about 2000 to about 3000 base pairs, at least about 2000 base pairs, at least about 4000 base pairs or at least about 6000 base pairs. In a third embodiment, the spacer element may not express any genes.

In a fourth embodiment, the spacer element may express one or more genes advantageous for adenovirus or protein production such as, but not limited to, anti-apoptotic genes, growth promoting genes, kinases and selectable markers. Anti-apoptotic genes include, but are not limited to, CrmA and Bcl-2. Growth promoting genes include, but are not limited to, dominant negative double-stranded RNA-dependent protein kinase (PKR), adenoviral VA gene, SV40 T-antigen gene and cytokines. In an advantageous embodiment, the gene is dominant negative double-stranded RNA-dependent protein kinase (PKR) or adenoviral VA gene. More preferably the adenoviral VA gene comprises a mutated internal promoter, such that the transcription specificity is changed from polymerase III to polymerase II. The expression level of the modified VA gene can be modulated by the use of an inducible polymerase II promoter system such as, but not limited to the tetracycline repressor or metallothionein promoter systems. Selectable markers include, but are not limited to, neomycin, puromycin, zeomycin, bleomycin and ABC transporter (such as ABCG2, see, e.g., International Patent Publication WO 03/035685). The one or more genes may be inserted into the spacer element randomly or may be inserted into the spacer element by site directed integration.

There are several embodiments of the promoters. In a first embodiment, the expression of one or more genes may be driven by a constitutive promoter, an inducible promoter or a regulatable promoter. A constitutive promoter may be selected from the group consisting of CMV, RSV, SV40, PKG and TK. An inducible promoter or regulatable promoter may be selected from the group consisting of a metallothionein promoter or a tetracycline repressor ("tetR") system. Advantageously, the promoter is a metallothionein promoter, preferably a sheep metallothionein promoter 1 a gene promoter.

The present invention encompasses methods of repressing undesirable homologous recombination or their effects in a mammalian cell comprising inserting a non-homologous spacer element into an adenovirus nucleotide sequence.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings, in which:
FIG. 1 depicts RCA generation in 293 cells,
FIG. 2 depicts RCA free vector production in PER.C6 cell line,
FIG. 3 depicts VLI-293 cells,
FIG. 4 depicts potential spacer configurations, including several expression cassettes, regulatory elements (poly A, enhancers, promoters, insulators), regulatable promoters (e.g. sMT promoter, tetR), anti-apoptotic genes (e.g. CrmA, Bcl-2), growth promoting genes (e.g. dominant negative PKR, cytokines), selectable markers (e.g. neomycin, puromycin, ABCG2) and secondary integration target sites (e.g.lox, frt, attB phiC31 sequences)
FIG. 5 depicts an E1 B / pIX promoter region,
FIG. 6 depicts a spacer configuration,
FIG. 7 depicts RT-PCR analysis of DHFR expression where lane 1 is the 1kb+ marker, lane 2 is mock transfected 293 cells -zn, lane 3 is mock transfected 293 cells +zn, lane 4 is pMT010/a+ transfected 293 cells -zn, lane 5 is pMT010/a+ transfected 293 cells - zn, lane 6 is pGL3-TKpr-DHFR-TKpA transfected 293 cells, lane 7 is pRc-Ad5-ec1-3-lox transfected 293 cells and lane 8 is a dH2O control,
FIG. 8 depicts generation of test vector for pRcCMV-IVS-Cre,
FIG. 9 depicts an analysis of Cre recombinase activity with the top row demonstrating cells under visible light, and the bottom row demonstrating cells under UV for fluorescence for detection of eGFP,
FIG. 10 depicts analysis of TK functionality and
FIG. 11 depicts generation of the pRc-Spacer circular plasmid.

### DETAILED DESCRIPTION

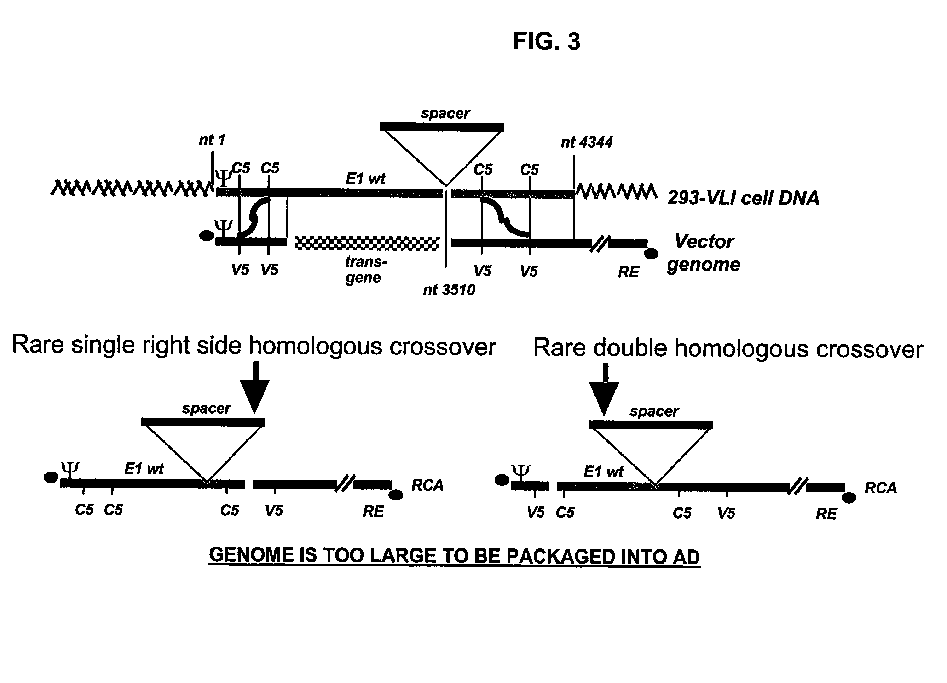

The present invention is based, in part, upon a novel cell-line based on 293 cells which represses undesirable homologous recombination events or their effects by the use of a large non-homologous spacer element(s). See, e.g., FIG. 3.

In accordance with the present invention, there may be employed conventional molecular biology, microbiology, and recombinant DNA techniques within the skill of the art. Such techniques are explained fully in the literature. See, e.g., Maniatis, Fritsch & Sambrook, "Molecular Cloning: A Laboratory Manual (1982); "DNA Cloning: A Practical Approach," Volumes I and II (D.N. Glover ed. 1985); "Oligonucleotide Synthesis" (M.J. Gait ed. 1984); "Nucleic Acid Hybridization" [B.D. Hames & S.J. Higgins eds. (1985)]; "Transcription and Translation" [B.D. Hames & S.J. Higgins eds. (1984)]; "Animal Cell Culture" [R.I. Freshney, ed. (1986)]; "Immobilized Cells And Enzymes" [IRL Press, (1986)]; B. Perbal, "A Practical Guide To Molecular Cloning" (1984). Therefore, if appearing herein, the following terms shall have the terminology set out below.

A "DNA molecule" refers to the polymeric form of deoxyribonucleotides (adenine, guanine, thymine, or cytosine) in its either single stranded form, or a double-stranded helix. This term refers only to the primary and secondary structure of the molecule, and does not limit it to any particular tertiary forms. Thus, this term includes double-stranded DNA found, inter alia, in linear DNA molecules (e.g., restriction fragments), viruses, plasmids, and chromosomes. In discussing the structure herein according to the normal convention of giving only the sequence in the 5' to 3' direction along the nontranscribed strand of DNA (i.e., the strand having a sequence homologous to the mRNA).

A "vector" is a replicon, such as plasmid, phage or cosmid, to which another DNA segment may be attached so as to bring about the replication of the attached segment. A "replicon" is any genetic element (e.g., plasmid, chromosome, virus) that functions as an autonomous unit of DNA replication in vivo; i.e., capable of replication under its own control. An "origin of replication" refers to those DNA sequences that participate in DNA synthesis. An "expression control sequence" is a DNA sequence that controls and regulates the transcription and translation of another DNA sequence. A coding sequence is "operably linked" and "under the control" of transcriptional and translational control sequences in a cell when RNA polymerase transcribes the coding sequence into mRNA, which is then translated into the protein encoded by the coding sequence.

In general, expression vectors containing promoter sequences which facilitate the efficient transcription and translation of the inserted DNA fragment are used in connection with the host. The expression vector typically contains an origin of replication, promoter(s), terminator(s), as well as specific genes which are capable of providing phenotypic selection in transformed cells. The transformed hosts can be fermented and cultured according to means known in the art to achieve optimal cell growth.

A DNA "coding sequence" is a double-stranded DNA sequence which is transcribed and translated into a polypeptide in vivo when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. A polyadenylation signal and transcription termination sequence will usually be located 3' to the coding sequence. A "cDNA" is defined as copy-DNA or complementary-DNA, and is a product of a reverse transcription reaction from an mRNA transcript.

Transcriptional and translational control sequences are DNA or RNA regulatory sequences, such as promoters, enhancers, polyadenylation signals, terminators, and the like, that provide for the expression of a coding sequence in a host cell. A "cis-element" is a nucleotide sequence, also termed a "consensus sequence" or "motif", that interacts with other proteins which can upregulate or downregulate expression of a specific gene locus. A "signal sequence" can also be included with the coding sequence. This sequence encodes a signal peptide, N-terminal to the polypeptide, that communicates to the host cell and directs the polypeptide to the appropriate cellular location. Signal sequences can be found associated with a variety of proteins native to prokaryotes and eukaryotes.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence is a transcription initiation site, as well as protein binding domains (consensus sequences) responsible for the binding of RNA polymerase. Eukaryotic promoters often, but not always, contain "TATA" boxes and "CAT" boxes. Prokaryotic promoters contain Shine-Dalgamo sequences in addition to the -10 and -35 consensus sequences.

The term "oligonucleotide" is defined as a molecule comprised of two or more deoxyribonucleotides, preferably more than three. Its exact size will depend upon many factors which, in turn, depend upon the ultimate function and use of the oligonucleotide. The term "primer" as used herein refers to an oligonucleotide, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and use for the method. For example, for diagnostic applications, depending on the complexity of the target sequence, the oligonucleotide primer typically contains 15-25 or more nucleotides, although it may contain fewer nucleotides.

The primers herein are selected to be "substantially" complementary to different strands of a particular target DNA or RNA sequence. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence to hybridize therewith and thereby form the template for the synthesis of the extension product.

As used herein, the terms "restriction endonucleases" and "restriction enzymes" refer to enzymes which cut double-stranded DNA at or near a specific nucleotide sequence.

"Recombinant DNA technology" refers to techniques for uniting two heterologous DNA molecules, usually as a result of in vitro ligation of DNAs from different organisms. Recombinant DNA molecules are commonly produced by experiments in genetic engineering. Synonymous terms include "gene splicing", "molecular cloning" and "genetic engineering". The product of these manipulations results in a "recombinant" or "recombinant molecule".

A cell has been "transformed" or "transfected" with exogenous or heterologous DNA when such DNA has been introduced inside the cell. The transforming DNA may or may not be integrated (covalently linked) into the genome of the cell. In prokaryotes, yeast, and mammalian cells for example, the transforming DNA may be maintained on an episomal element such as a vector or plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or ancestor by mitosis. A "cell line" is a clone of a primary cell that is capable of stable growth in vitro for many generations. An organism, such as a plant or animal, that has been transformed with exogenous DNA is termed "transgenic".

Two DNA sequences are "substantially homologous" when at least about 75% (preferably at least about 80%, and most preferably at least about 90% or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, e.g., Maniatis et al., supra; DNA Cloning, Vols. I & II, supra; Nucleic Acid Hybridization, supra.

A "heterologous" region of the DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. In another example, the coding sequence is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein. For example, a polynucleotide may be placed by genetic engineering techniques into a plasmid or vector derived from a different source and be a heterologous polynucleotide. A promoter removed from its native coding sequence and operatively linked to a coding sequence other than the native sequence is a heterologous promoter.

As used herein, "fragment" or "portion" as applied to a gene or a polypeptide, will ordinarily be at least 10 residues, more typically at least 20 residues, and preferably at least 30 (e.g., 50) residues in length, but less than the entire, intact sequence. Fragments of these genes can be generated by methods known to those skilled in the art, e.g., by restriction digestion of naturally occurring or recombinant genes, by recombinant DNA techniques using a vector that encodes a defined fragment or gene, or by chemical synthesis.

A standard northern blot assay can be used to ascertain the relative amounts of mRNA in a cell or tissue in accordance with conventional northern hybridization techniques known to those persons of ordinary skill in the art. Alternatively, a standard Southern blot assay may be used to confirm the presence and the copy number of the gene of interest in accordance with conventional Southern hybridization techniques known to those of ordinary skill in the art. Both the northern blot and Southern blot use a hybridization probe, e.g. radiolabelled cDNA or oligonucleotide of at least 20 (preferably at least 30, more preferably at least 50, and most preferably at least 100 consecutive nucleotides in length). The DNA hybridization probe can be labelled by any of the many different methods known to those skilled in this art.

Hybridization reactions can be performed under conditions of different "stringency." Conditions that increase stringency of a hybridization reaction are well known. See for examples, "Molecular Cloning: A Laboratory Manual", second edition (Sambrook et al. 1989). Examples of relevant conditions include (in order of increasing stringency): incubation temperatures of 25°C, 37°C, 50°C, and 68°C; buffer concentrations of 10 x SSC, 6 x SSC, 1 x SSC, 0.1 x SSC (where SSC is 0.15 M NaCl and 15 mM citrate buffer) and their equivalent using other buffer systems; formamide concentrations of 0%, 25%, 50%, and 75%; incubation times from 5 minutes to 24 hours; 1, 2 or more washing steps; wash incubation times of 1, 2, or 15 minutes; and wash solutions of 6 x SSC, 1 x SSC, 0.1 x SSC, or deionized water.

The labels most commonly employed for these studies are radioactive elements, enzymes, chemicals which fluoresce when exposed to ultraviolet light, and others. A number of fluorescent materials are known and can be utilized as labels. These include, for example, fluorescein, rhodamine, auramine, Texas Red, AMCA blue and Lucifer Yellow. A particular detecting material is anti-rabbit antibody prepared in goats and conjugated with fluorescein through an isothiocyanate. Proteins can also be labeled with a radioactive element or with an enzyme. The radioactive label can be detected by any of the currently available counting procedures. The preferred isotope may be selected from ³H, ¹⁴C, ³²P, ³⁵S, ³⁶Cl, ⁵¹Cr , ⁵⁷Co, ⁵⁸Co, ⁵⁹Fe, ⁹⁰Y, ¹²⁵I, ¹³¹I, and ¹⁸⁶Re.

Enzyme labels are likewise useful, and can be detected by any of the presently utilized colorimetric, spectrophotometric, fluorospectrophotometric, amperometric or gasometric techniques. The enzyme is conjugated to the selected particle by reaction with bridging molecules such as carbodiimides, diisocyanates, glutaraldehyde and the like. Many enzymes which can be used in these procedures are known and can be utilized. The preferred are peroxidase, β-glucuronidase, β-D-glucosidase, β-D-galactosidase, urease, glucose oxidase plus peroxidase and alkaline phosphatase. U.S. Pat. Nos. 3,654,090, 3,850,752, and 4,016,043 are referred to by way of example for their disclosure of alternate labeling material and methods.

The term "exogenous gene," as it is used herein, refers to any gene in the spacer element within the adenovirus sequence. The gene includes a nucleic acid sequence encoding a gene product operably linked to a promoter. For example, the gene can comprise a non-native nucleic acid sequence encoding a gene product operably linked to a native promoter, or a native nucleic acid sequence encoding a gene product operably linked to a non-native promoter or in a non-native location. It should be appreciated that the exogenous gene can be any gene encoding an RNA or protein of interest to the skilled artisan. Therapeutic genes, genes encoding a protein that is to be studied in vitro and/or in vivo, antisense nucleic acids, and modified viral genes are illustrative of possible exogenous genes.

The novel cell-line may be based on 293 cells and is referred to as "293-VLI cells". 293-VLI cells do not eliminate the overlap between the cell-line and adenoviral vector sequences, but repress undesirable homologous recombination events or their effects by the use of a large non-homologous spacer element(s). In a preferred embodiment the spacer is inserted by site directed integration at a specific site into the already existing Ad sequence in 293 cells.

The specific site in the adenovirus nucleotide sequence may be after the end of the early region 1B ("E1B") transcription unit but in front of the protein IX ("pIX") transcription unit, after the inverted terminal repeat ("ITR") and packaging sequences but before the early region 1A ("E1A") transcription start site or after the E1A sequences but before the E1B sequences, or at other regions that do not disrupt their function.

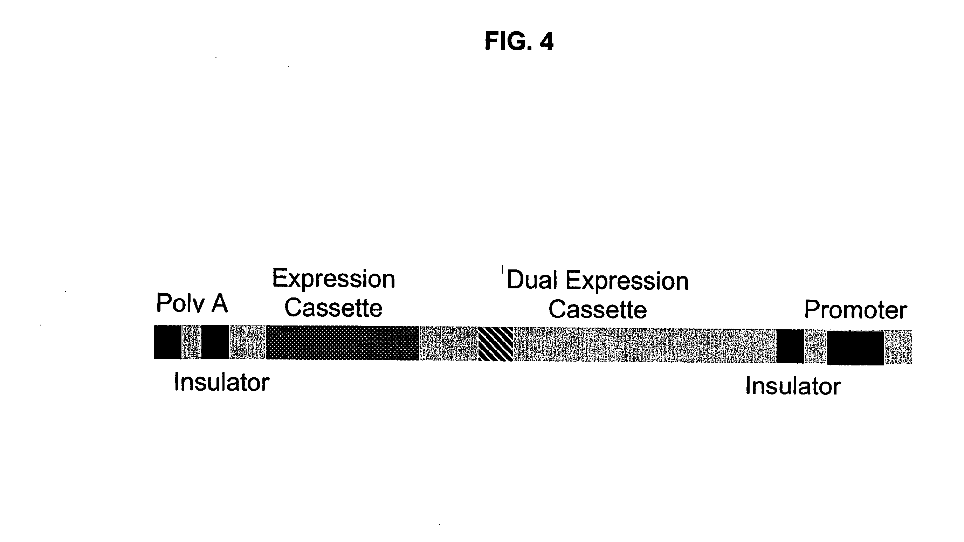

Potential spacer configurations are depicted in FIG. 4.

The spacer element may comprise one or more regulatory elements such as, but not limited to, promoters, enhancers, insulators, polyadenylation and termination signals.

The spacer element may comprise site specific recombination element(s) such as, but not limited to, the lox, frt and attB sites used by the CRE, FLPe and phiC31 systems respectively. Insertion of spacer element(s) can be also accomplished by the use of transposable element(s) as such as, but not limited to, Sleeping Beauty transposase.

A preferred site of insertion is after nucleotide 3510 at the end of the E1B transcription unit, but in front of the pIX transcription unit. Additional sites of insertions could create further reduction in the probability of RCA generation. One site of insertion is after the ITR and packaging sequences but before the E1A transcriptional start site. Furthermore, another site of insertion is after the E1A sequences, but before the E1B sequences. In these insertions some of the regulatory elements (promoters, enhancers, poly A or termination signals) may be provided by the spacer element.

The spacer may serve two functions. First, the size of a non-homologous spacer radically represses homologous recombination between direct repeats as the size increases to ∼ 2-3000 base pairs (bp) (see, e.g., Perez et al., 2005, Biotechniques 39:109-15). Second, the size limit on adenovirus packaging is 105% (see, e.g., Bett et al., 1993, J Virol 67:5911-21), therefore even in the rare event of homologous recombination between the overlapping Ad sequences, the produced Ad would be too large to be efficiently packaged and propagated. Taking these points into consideration and knowing that the Ad genome is ∼ 36,000 bp, the preferred size of the spacer should not be smaller than 2000 bp, but preferably should be larger than 4000 bp and even more preferably should be larger than 6000 bp.

In one embodiment, the spacer sequence is inert (i.e. not expressing any transcripts).

In a preferred embodiment the spacer sequence contain several expression cassettes expressing different genes that are advantageous for Ad or protein production. The spacer element expresses one or more genes advantageous for adenovirus or protein production such as, but not limited to, anti-apoptotic genes, growth promoting genes, kinases and selectable markers. Anti-apoptotic genes include, but are not limited to, CrmA and Bcl-2. Growth promoting genes include, but are not limited to, dominant negative double-stranded RNA-dependent protein kinase (PKR), adenoviral VA gene, SV40 T-antigen gene and cytokines. In an advantageous embodiment, the gene is dominant negative double-stranded RNA-dependent protein kinase (PKR) or modified adenoviral VA gene.

The double-stranded (ds) RNA-dependent protein kinase (PKR), also termed eukaryotic translation initiation factor 2-alpha kinase 2 (EIF2AK2), (also termed DAI, p68, DsI, Pl/eIF-2, and PRKR); GenBenk Accession: NM_002759, has a central role in the mechanisms employed by the cell to counteract virus attacks (see, e.g., Proud, 1995, Trends Biochem Sci 20:241-6). PKR phosphorylates the translation initiation factor eIF-2 and inhibits its activity and so shutting down the translational machinery of the cell. However, PKR plays a role in normal control of cell growth and differentiation and so the amounts of phosphorylated and un-phosphorylated eIF-2 are in equilibrium. By shifting this equilibrium towards the un-phosphorylated form, the maximum translational capacity (i.e. protein production) of the cell could be harvested. There are several trans-dominant negative mutants of PKR (dnPKR) has been identified. It has been also demonstrated, that expression of dnPKR in a cell could increase protein production transiently (see, e.g., Donze et al., 1999, 322-9. Virology 256). It also has been demonstrated that retrovirus production can be increased by the use ofPKR inhibitors (see, e.g., Pernod et al., 2004, Biotechniques 36:576-8, 580).

It has been also demonstrated, that transient expression of the adenoviral VA gene in a cell could increase protein production transiently (Akusjarvi et. al. (1987) Mol. Cel. Biol. 7, 549; Kaufman and Murtha (1987) Mol. Cel. Biol. 7, 1568). The permanent expression of VA gene in a cell line could be deleterious, and might need to be regulated. The adenoviral VA gene is transcribed by polymerse III through internal promoter sequences. Mutating these internal promoter sequences, such that the transcription specificity is changed from polymerase III to polymerase II allows for the operatively linking a regulatable polymerase II promoter. The expression level of the modified VA gene can be modulated by the use of an inducible polymerase II promoter system such as, but not limited to the tetracycline repressor or metallothionein promoter systems.

Selectable markers include, but are not limited to, neomycin, puromycin, zeomycin, bleomycin and ABC transporter. The one or more genes may be inserted into the spacer element randomly or may be inserted into the spacer element by site directed integration.

These advantageous gene sequences could be inserted into the cell-line genome randomly without the use of site directed integration. However, the preferred method would be site directed integration through the incorporation of these gene sequences into the spacer element. This method has the great advantage of predetermining the exact configuration, position and number of transcription elements. This greatly simplifies the later characterization of the cell-line according to regulatory requirements.

The invention also provides a system comprising the inventive cell and a replication-deficient adenoviral vector comprising an adenoviral genome deficient in the at least one essential gene function of the one or more regions (i.e., a replication-deficient adenoviral vector comprising the deficiencies complemented for by the inventive cell). The inventive cell complements the E1A and E1B deficiencies; other adenovirus vector deficiencies can be complemented or supplemented through the incorporation of the adenovirus genes into the cell genome. In the most preferred embodiment the adenovirus complementing genes that are missing or poorly expressed in the vector are incorporated into the spacer element(s) (e.g., E4, E4-ORF6, E2A, E2B, pIX, fiber, penton base, hexon). The invention further provides a method of propagating a replication-deficient adenoviral vector. The method comprises providing a cell of the invention, introducing the replication-deficient adenoviral vector into the cell, wherein the replication-deficient adenoviral vector comprises an adenoviral genome deficient in at least one essential gene function of one or more regions, and maintaining the cell (e.g., under conditions suitable for adenoviral propagation) to propagate the adenoviral vector.

The adenoviral vector is deficient in at least one gene function (of the adenoviral genome) required for viral propagation (i.e., an adenoviral essential gene function), thereby resulting in a "replication-deficient" adenoviral vector. The adenoviral vector is deficient in the one or more adenoviral essential gene functions complemented for by the inventive cell to allow for propagation of the replication-deficient adenoviral vector when present in the cell.

Preferably, the adenoviral vector is deficient in at least one essential gene function of the E1 region, e.g., the E1a region and/or the E1b region, of the adenoviral genome that is required for viral replication. The recombinant adenovirus also can have a mutation in the major late promoter (MLP), as discussed in International Patent Application WO 00/00628. More preferably, the vector is deficient in at least one essential gene function of the E1 region and at least part of the nonessential E3 region (e.g., an Xba I deletion of the E3 region). The adenoviral vector can be "multiply deficient," meaning that the adenoviral vector is deficient in one or more essential gene functions in each of two or more regions of the adenoviral genome. For example, the aforementioned E1-deficient or E1-, E3-deficient adenoviral vector can be further deficient in at least one essential gene of the E4 region and/or at least one essential gene of the E2 region (e.g., the E2A region and/or E2B region). Adenoviral vectors deleted of the entire E4 region can elicit lower host immune responses. Examples of suitable adenoviral vectors include adenoviral vectors that lack (a) all or part of the E1 region and all or part of the E2 region, (b) all or part of the E1 region, all or part of the E2 region, and all or part of the E3 region, (c) all or part of the E1 region, all or part of the E2 region, all or part of the E3 region, and all or part of the E4 region, (d) at least part of the E1a region, at least part of the E1b region, at least part of the E2a region, and at least part of the E3 region, (e) at least part of the E1 region, at least part of the E3 region, and at least part of the E4 region, and (f) all essential adenoviral gene products (e.g., adenoviral amplicons comprising ITRs and the packaging signal only). The adenoviral vector can contain a wild type pIX gene. Alternatively, although not preferably, the adenoviral vector also can contain a pIX gene that has been modified by mutation, deletion, or any suitable DNA modification procedure. In any of these embodiments the adenoviral sequences may lie within their normal context or be relocated to other regions of the vector or be in an alternative orientation. They may have been genetically modified to exploit codon degeneracy while maintaining the function of one or more encoded viral proteins.

The replication-deficient adenoviral vector can be generated by using any species, strain, subtype, or mixture of species, strains, or subtypes, of an adenovirus or a chimeric adenovirus as the source of vector DNA. The adenoviral vector can be any adenoviral vector capable of growth in a cell, which is in some significant part (although not necessarily substantially) derived from or based upon the genome of an adenovirus. The adenoviral vector preferably comprises an adenoviral genome of a wild-type adenovirus of group C, especially of serotype (i.e., Ad5). Adenoviral vectors are well known in the art and are described in, for example, U.S. Pat. Nos. 5,559,099, 5,712,136, 5,731,190, 5,837,511, 5,846,782, 5,851,806, 5,962,311, 5,965,541, 5,981,225, 5,994,106, 6,020,191, and 6,113,913, International Patent Applications WO 95/34671, WO 97/21826, and WO 00/00628, and Thomas Shenk, "Adenoviridae and their Replication," and M. S. Horwitz, "Adenoviruses," Chapters 67 and 68, respectively, in Virology, B. N. Fields et al., eds., 3d ed., Raven Press, Ltd., New York (1996).

The construction of adenoviral vectors is well understood in the art and involves the use of standard molecular biological techniques, such as those described in, for example, Sambrook et al., supra, Watson et al., supra, Ausubel et al., supra, and other references mentioned herein. Moreover, adenoviral vectors can be constructed and/or purified using the methods set forth, for example, in U.S. Pat. No. 5,965,358 and International Patent Applications WO 98/56937, WO 99/15686, and WO 99/54441.

When a cell is used to propagate a replication-deficient adenoviral vector, it is desirable to avoid a recombination event between the cellular genome (of the cell) and the adenoviral genome (of the adenoviral vector) that would result in the generation of a replication-competent adenovirus (RCA). As such, there is preferably insufficient overlap between the genome of the cell and the replication-deficient adenoviral vector genome to mediate a recombination event sufficient to result in a replication-competent adenovirus. If overlap exists, the overlapping sequences desirably are predominantly located in the nucleic acid flanking the coding region of the complementation factor (the "trans-complementing region") in the cellular genome and the nucleotide sequences adjacent to the missing region(s) of the adenoviral genome. Ideally, there is no sequence overlap between the cellular genome and the adenoviral vector genome. However, it is acceptable that partial overlap exists between the sequences of the cellular genome and the adenoviral vector genome on one side of the trans-complementing region. In such an event, the region of homology preferably is contiguous with the trans-complementing region. The generation of RCA desirably is diminished such that (a) the cell produces less than about one replication-competent adenoviral vector for at least about 20 passages after infection with the adenoviral vector, (b) the cell produces less than about one replication-competent adenoviral vector in a period of about 36 hours post-infection, (c) the cell produces less than about one replication-competent adenoviral vector per 1 x 10¹⁰ total viral particles (preferably 1 x 10¹¹ total viral particles, more preferably 1 x 10¹² total viral particles, and most preferably 1 x 10¹³ total viral particles), or any combination of (a)-(c). Optimally, the amount of overlap between the cellular genome and the adenoviral genome (i.e., the genome of the adenoviral vector being propagated in the cell) is insufficient to mediate a homologous recombination event that results in a replication-competent adenoviral vector such that replication-competent adenoviruses are eliminated from the vector stocks resulting from propagation of the replication-deficient adenoviral vector in the cell. Virus growth yield and virus plaque formation have been previously described (see, e.g., Burlseson et al., Virology: a Laboratory Manual, Academic Press Inc. (1992)), and measuring RCA as a function of plaque forming units is described in U.S. Pat. 5,994,106.

Alternatively, the adenoviral vector is preferably conditionally replication deficient in at least one gene function required for viral replication in specific cells or tissues. Preferably, the adenoviral vector is deleted in at least one essential gene of the E1 region of the adenoviral genome, particularly the E1a region, more preferably, the vector is deficient in the retinoblastoma (Rb) binding site as described in U.S. Pat. No. 6,824,771.

It should be appreciated that the deletion of different regions of the adenoviral gene transfer vector can alter the immune response of the mammal, in particular, deletion of different regions can reduce the inflammatory response generated by the adenoviral gene transfer vector. Furthermore, the adenoviral gene transfer vector's coat protein can be modified so as to decrease the adenoviral gene transfer vector's ability or inability to be recognized by a neutralizing antibody directed against the wild-type coat protein, as described in International Patent Application WO 98/40509. Other suitable modifications to the adenoviral gene transfer vector are described in U.S. Pat. Nos. 5,559,099; 5,731,190; 5,712,136; and 5,846,782 and International Patent Applications WO 97/20051, WO 98/07877, and WO 98/54346.

Methods for making and/or administering a vector or recombinants or plasmid for expression of gene products of genes of the invention either *in vivo* or *in vitro* can be any desired method, e.g., a method which is by or analogous to the methods disclosed in, or disclosed in documents cited in: U.S. Patent Nos. 4,603,112; 4,769,330; 4,394,448; 4,722,848; 4,745,051; 4,769,331; 4,945,050; 5,494,807; 5,514,375; 5,744,140; 5,744,141; 5,756,103; 5,762,938; 5,766,599; 5,990,091; 5,174,993; 5,505,941; 5,338,683; 5,494,807; 5,591,639; 5,589,466; 5,677,178; 5,591,439; 5,552,143; 5,580,859; 6,130,066; 6,004,777; 6,130,066; 6,497,883; 6,464,984; 6,451,770; 6,391,314; 6,387,376; 6,376,473; 6,368,603; 6,348,196; 6,306,400; 6,228,846; 6,221,362; 6,217,883; 6,207,166; 6,207,165; 6,159,477; 6,153,199; 6,090,393; 6,074,649; 6,045,803; 6,033,670; 6,485,729; 6,103,526; 6,224,882; 6,312,682; 6,348,450 and 6; 312,683; U.S. patent application Serial No. 920,197, filed October 16,1986; WO 90/01543; W091/11525; WO 94/16716; WO 96/39491; WO 98/33510; EP 265785; EP 0 370 573; Andreansky et al., Proc. Natl. Acad. Sci. USA 1996;93:11313-11318; Ballay et al., EMBO J. 1993;4:3861-65; Felgner et al., J. Biol. Chem. 1994;269:2550-2561; Frolov et al., Proc. Natl. Acad. Sci. USA 1996;93:11371-11377; Graham, Tibtech 1990;8:85-87; Grunhaus et al., Sem. Virol. 1992;3:237-52; Ju et al., Diabetologia 1998;41:736-739; Kitson et al., J. Virol. 1991;65:3068-3075; McClements et al., Proc. Natl. Acad. Sci. USA 1996;93:11414-11420; Moss, Proc. Natl. Acad. Sci. USA 1996;93:11341-11348; Paoletti, Proc. Natl. Acad. Sci. USA 1996;93:11349-11353; Pennock et al., Mol. Cell. Biol. 1984;4:399-406; Richardson (Ed), Methods in Molecular Biology 1995;39, "Baculovirus Expression Protocols," Humana Press Inc.; Smith et al. (1983) Mol. Cell. Biol. 1983;3:2156-2165; Robertson et al., Proc. Natl. Acad. Sci. USA 1996;93:11334-11340; Robinson et al., Sem. Immunol. 1997;9:271; and Roizman, Proc. Natl. Acad. Sci. USA 1996;93:11307-11312.

There are several embodiments of the promoters. In a first embodiment, the expression of one or more genes is driven by a constitutive promoter, an inducible promoter or a regulatable promoter. A constitutive promoter is selected from the group consisting of CMV, RSV, SV40, PKG and TK. An inducible promoter or regulatable promoter is selected from the group consisting of a metallothionein promoter or a tetracycline repressor ("tetR") system. Advantageously, the promoter is a metallothionein promoter, preferably a sheep metallothionein promoter la gene promoter.

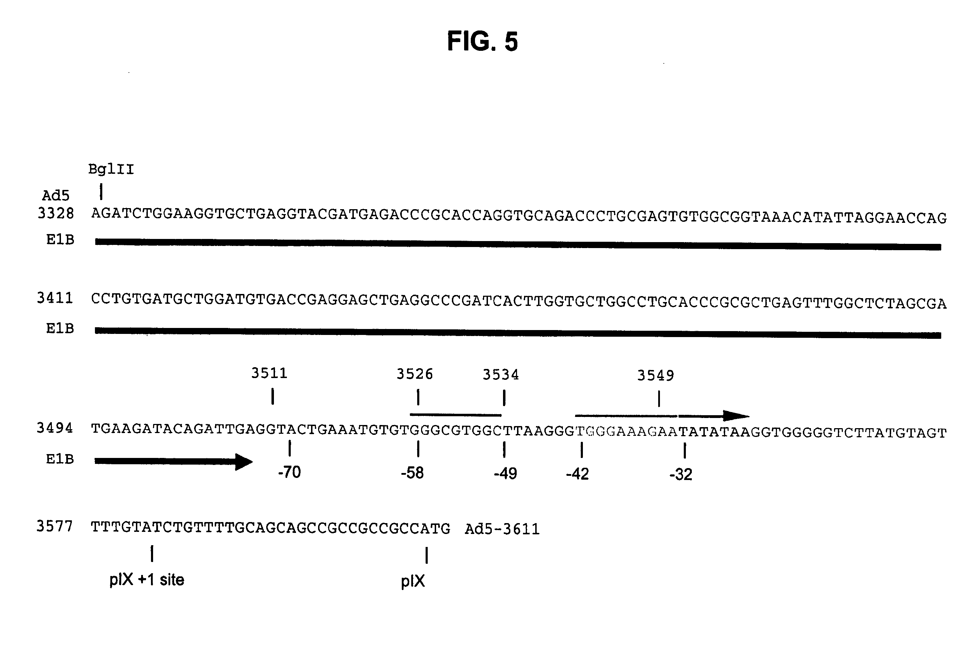

FIG. 5 depicts an E1B / pIX promoter region.

Permanent incorporation of a nucleic acid sequence into the cell genome, which upon expression produces dnPKR protein or adenovirus VA RNA can have beneficial effects on the production of a specific protein or adenovirus vector. It is possible that dnPKR or VA transcripts are produced from a constitutive promoter persistently without causing any deleterious effects to the cell line, however this is unlikely. Constitutive expression of dnPKR or VA is likely impossible as it would be deleterious to the cell-line stability and production features. Therefore, the incorporation of a regulatable system is preferred.

There have been many systems described in the prior art to regulate gene expression of a polymerase II transcribed gene. One of the possibilities is to use a repressible system like the use of the tetracyclin repressor (tetR) system described in U.S. Patent No. 5,972,650. However, the preferred configuration is a simple inducible system. A preferred system is the sheep metallothionein la gene (sMT-Ia) promoter, which has a very low basal level and is inducible with zinc (see, e.g., Peterson et al., 1986, Eur J Biochem 160:579-85 and U.S. Patent Nos. 5,851,806, 5,994,106 and 6,482,616). To use these polymerase II systems with the adenoviral VA gene its internal promoter sequences would need to be mutated to inactivate polymerase III transcription.

Polynucleotides comprising a desired sequence can be inserted into a suitable cloning or expression vector, and the vector in turn can be introduced into a suitable host cell for replication and amplification. Polynucleotides can be introduced into host cells by any means known in the art. The vectors containing the polynucleotides of interest can be introduced into the host cell by any of a number of appropriate means, including direct uptake, endocytosis, transfection, f-mating, electroporation, transfection employing calcium chloride, rubidium chloride, calcium phosphate, DEAE-dextran, or other substances; microprojectile bombardment; lipofection; and infection (where the vector is infectious, for instance, a retroviral vector). The choice of introducing vectors or polynucleotides will often depend on features of the host cell.

In view of the above, the method can further comprise subsequently repeating the administration of an adenoviral gene transfer vector comprising the exogenous gene encoding the gene product and/or a replication competent Ad vector with or without vector comprising the exogenous gene encoding the gene product to the appropriate tissue of the animal.

Thus, the inventive virions can be targeted to cells within any organ or system, including, for example, respiratory system (e.g., trachea, upper airways, lower airways, alveoli), nervous system and sensory organs (e.g., skin, ear, nasal, tongue, eye), digestive system (e.g., oral epithelium and sensory organs, salivary glands, stomach, small intestines/duodenum, colon, gall bladder, pancreas, rectum), muscular system (e.g., skeletal muscle, connective tissue, tendons), skeletal system (e.g., joints (synovial cells), osteoclasts, osteoblasts, etc.), immune system (e.g., bone marrow, stem cells, spleen, thymus, lymphatic system, etc.), circulatory system (e.g., muscles, connective tissue, and/or endothelia of the arteries, veins, capillaries, etc.), reproductive system (e.g., testes, prostate, uterus, ovaries), urinary system (e.g., bladder, kidney, urethra), endocrine or exocrine glands (e.g., breasts, adrenal glands, pituitary glands), etc or delivered systemically. These adenoviral vectors are capable of delivering gene products with high efficiency and specificity to cells expressing receptors which recognize the ligand component of the fiber-fibritin-ligand chimera. A person having ordinary skill in this art would recognize that one may exploit a wide variety of genes encoding e.g. receptor ligands or antibody fragments which specifically recognize cell surface proteins unique to a particular cell type to be targeted.

The invention further encompasses a method for administrating the adenovirus of the present invention propagated in the cell-line of the present invention to a subject in need thereof which may comprise administering to the subject in need thereof a therapeutically effective amount of the adenovirus described herein wherein the non-native amino acid targets the tumor cell such that the adenovirus infects the target cells.

The present invention can be practiced with any suitable animal, preferably the present invention is practiced with a mammal, more preferably, a human. Additionally, the adenoviral vector can be a gene transfer vector or a replication competent vector and can be administered, e.g., once, twice, or more, to any suitable tissue or delivered systemically to the animal. Systemic administration can be accomplished through intravenous injection, either bolus or continuous, or any other suitable method.

After subsequent administration of the adenoviral gene transfer vector comprising an exogenous gene, production of the gene product in the tissue of the animal is desirably at least 1% of (such as at least 10% of, preferably at least 50% of, more preferably at least 80% of, and most preferably, the same as or substantially the same as) production of the gene product after initial administration of the same adenoviral gene transfer vector containing the exogenous gene. Methods for comparing the amount of gene product produced in the tissue of administration are known in the art. The comparison can be made at the same time after the initial and subsequent administrations of the adenoviral gene transfer vector.

After subsequent administration of a replication competent adenoviral vector, replication of the vector in the tissue of the animal is desirably at least 1% of (such as at least 10% of, preferably at least 50% of, more preferably at least 80% of, and most preferably, the same as or substantially the same as) replication of the vector after initial administration. Methods for comparing the amount of adenovirus replication in the tissue of administration are known in the art. The comparison can be made at the same time after the initial and subsequent administrations of the adenoviral vector.

To facilitate the administration of adenoviral vectors, they can be formulated into suitable pharmaceutical compositions. Generally, such compositions include the active ingredient (i.e., the adenoviral vector) and a pharmacologically acceptable carrier. Such compositions can be suitable for delivery of the active ingredient to a patient for medical application, and can be manufactured in a manner that is itself known, e.g., by means of conventional mixing, dissolving, granulating, dragee-making, levigating, emulsifying, encapsulating, entrapping or lyophilizing processes.

Pharmaceutical compositions for use in accordance with the present invention can be formulated in a conventional manner using one or more pharmacologically or physiologically acceptable carriers comprising excipients, as well as optional auxiliaries, which facilitate processing of the active compounds into preparations, which can be used pharmaceutically. Proper formulation is dependent upon the route of administration chosen. Thus, for injection, the active ingredient can be formulated in aqueous solutions, preferably in physiologically compatible buffers. For transmucosal administration, penetrants appropriate to the barrier to be permeated are used in the formulation. Such penetrants are generally known in the art. For oral administration, the active ingredient can be combined with carriers suitable for inclusion into tablets, pills, dragees, capsules, liquids, gels, syrups, slurries, suspensions and the like. For administration by inhalation, the active ingredient is conveniently delivered in the form of an aerosol spray presentation from pressurized packs or a nebuliser, with the use of a suitable propellant. The active ingredient can be formulated for parenteral administration by injection, e.g., by bolus injection or continuous infusion. Such compositions can take such forms as suspensions, solutions or emulsions in oily or aqueous vehicles, and can contain formulatory agents such as suspending, stabilizing and/or dispersing agents. Other pharmacological excipients are known in the art.

Those of ordinary skill in the art can easily make a determination of the proper dosage of the adenoviral gene transfer vector. Generally, certain factors will impact the dosage that is administered; although the proper dosage is such that, in one context, the exogenous gene is expressed and the gene product is produced in the particular muscle of the mammal. Preferably, the dosage is sufficient to have a therapeutic and/or prophylactic effect on the animal. The dosage also will vary depending upon the exogenous gene to be administered. Specifically, the dosage will vary depending upon the particular muscle of administration, including the specific adenoviral vector, exogenous gene and/or promoter utilized. For purposes of considering the dose in terms of particle units (pu), also referred to as viral particles, it can be assumed that there are 100 particles per particle forming unit (pfu) (e.g., 1 x 10¹² pfu is equivalent to 1 x 10¹⁴ pu).

The present invention also encompasses methods of repressing homologous recombination in a mammalian cell comprising inserting a non-homologous spacer element into an adenovirus nucleotide sequence. The methods of repressing undesirable homologous recombination events in a mammalian cell or their effects, advantageously a human embryonic kidney or 293 cell, encompasses all embodiments of the novel cell line described herein.

The invention will now be further described by way of the following non-limiting examples.

### EXAMPLE

A highly efficient "base" expression cassette was designed and its level of expression tested with a luciferase gene for protein production capacity.

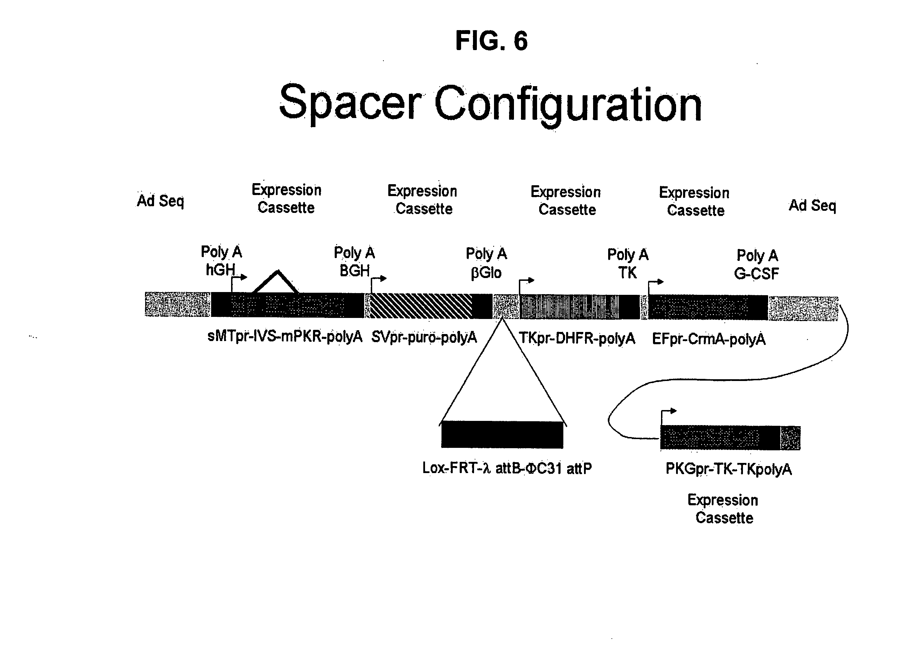

The spacer design is illustrated in FIG. 6. The spacer design consists of four expression cassettes (plus an extra poly A sequence terminating E1B) that inserted into the Ad chromosomal DNA sequence at nucleotide location 3511. The recombination plasmid includes the Ad homologous sequences and a TK expression cassette for negative selection. A short description of these cassettes is in Table 1.

**Table 1: Expression cassettes**

| **Expression cassette** | # | **Description** | **Purpose** | **∼Size (bp)** |
|---|---|---|---|---|
| sMTpr-IVS-mPKR-BGH | 1 | Regulatable promoter driving mPKR | Up regulation | 2000 |
| SVpr-puro-bGlo | 2 | SV40 promoter driving puromyocin | Selection | 1800 |
| TKpr-DHFR-TK | 3 | SV40 promoter driving DHFR | Amplification | 1500 |
| EFpr-CrmA-CSF | 4 | EFa promoter driving CrmA | Stabilization | 2000 |
| PKGpr-TK-TK | 5 | PKG promoter driving TK | Recombination selection | 2000 |
| **Total** | | **Spacer only** | **Minimum size** | **7300** |

**Table 2: Overview of cloning strategy**

| **Spacer Element** | **Backbone** | **Promoter** | **CDS** | **PolyA** | **pRc cloning** |
|---|---|---|---|---|---|
| **Ad5 sequence 2011-3510** | **pRcsMT-IVS-puro** | Generate PCR fragment from Ad5wt300 with blunt ends to clone into NruI site and hGH poly A to be incorporated at the end of E1B | | | To be cloned into NruI site |
| **sMT-PKR-BGHpolyA** | **pRcCMV-IVS-puro** | **sMT** From pMT010/A replace CMV Insert NruI/BstXI | **PKR-BD** From Origene PCR 1-243aa frag; Insert NotI/XbaI | **BGH** Already present | N/A |
| **SVpr-puro-bGlopolyA** | **pselect-puro** | **SV40** To insert into BspLU11I/AseI sites | **Puromycin** Already present | **bGlo** Already present | To replace the BamHI region - the inclusion of a multi- cloning site allows for further cloning in this plasmid |
| **Lox::frt::attB::** **phiC31::mcs** | **pselect-puro** | Generate fragment with **Lox::frt::attB::phiC31::mcs** Mcs = multicloning site Fragment generated with BamHI/EcorI ends to insert at 3' end of BGlo poly A | | | |
| **TKpr-DHFR-TKpolyA** | **pGL3-basic** | **TK** From pMEP4 | **mDHFR** From pMT010A HindIII/BglII | **TK** From pMEP4 | Cassette with AvrII/NheI ends inserted into NheI site |
| **hEF1pr-CrmA-G-CSFpolyA** | **pEF-BOS** On order | **hEF1** Already present | **crmA** Order from Planetgene Insert BstXI | **G-CSF** Already present | Cassette with AvrII/NheI ends inserted into NheI site |
| **Ad5 sequence 3511-5000** | **pRcsMT-IVS-puro** | Generate PCR fragment from Ad5wt300 with AvrII/NheI ends for pRc cloning | | | Cassette inserted into NheI site |
| **PKGpr-TK-TKpolyA** | **pNTK** | **PKG** Already present | **TK** Already present | **TK** Already present | Cassette inserted into AscI site |

Recombination sequences for the potential simple insertion of further expression cassettes for protein production are incorporated at a site between the puro and DHFR cassettes. This design allows for the efficient amplification of genomic sequences using methotrexate and puromycin. The insert incorporates the integration sequence elements: lox - frt - attB - phiC31.

### Generation of the spacer cassette in pRc backbone.

The pRc-Spacer contains all the cassettes described in Table 1 and illustrated in FIG. 6. pRcCMVpuro is starting backbone, and a synthetic intron (IVS) was inserted to improve expression of genes. In a sequential manner, various cassettes and additional sequences were generated. Thus far, three cassettes (1. sMTpr-IVS-mPKR-BGH, 2. SVpr-puro-bGlo and 3. TKpr-DHFR-TK), the 5' Ad5 sequence and the Lox region were inserted.

Briefly, for the first cassette a truncated version of the eukaryotic translation initiation factor 2-alpha kinase 2 (EIF2AK2), PKR-BD, which is the 1-243 aa portion of PKR, performed the best in the pRcCMV-IVS-puro backbone. At this point, the CMV promoter in pRcCMV-IVS-puro was replaced with the sheep metallothionein promoter from the pMT010/A+ vector which is induced in the presence of Zn. In front of this cassette, an Ad5 sequence from 2011-3510 a hGH polyA was inserted next to Ad5 nucleotide 3510 to prevent E1B read through.

A new puromycin cassette in pSelect-puro plasmid was created to include a SV40 promoter infront of the puromycin gene and utilized the plasmid BGlo polyA already present. The lox-phi sequence with a multi-cloning site was inserted following the BGlo polyA. This entire cassette was excised with BglII/BamHI and replaced the BamHI region in the pRc-IVS-puro backbone. The backbone containing both the Ad5 sequence and new puromycin cassette was named pRc-Ad5-ec1-2-lox. The puromycin cassette was confirmed to be functional by transforming E.coli and seeding onto agar-puro plates.

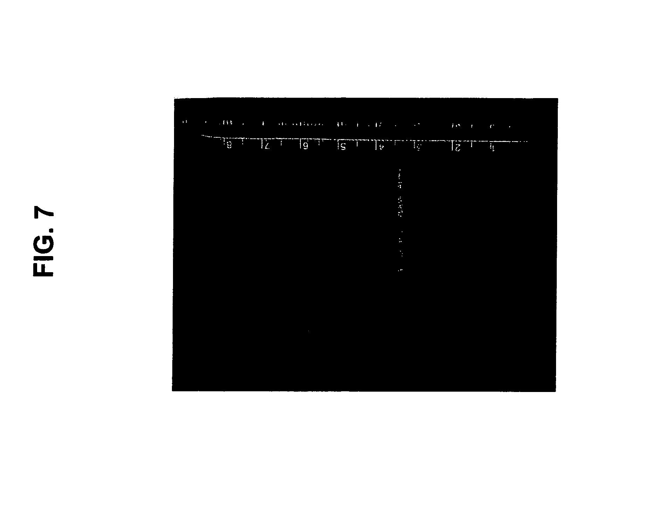

In addition the insertion of the TKpr-DHFR-TK cassette was completed, which was originally generated in the pGL3-basic backbone. Once all the pieces had been cloned into the pGL3 backbone, the TK promoter, murine DHFR fragment and TK polyA, and inserted an AvrII site, the cassette was excised from pGL3 with NheI/AvrII and inserted into the unique NheI site in the Lox-Phi-MCS fragment. This version of the plasmid is known as pRc-Ad5-ecl-3-lox. DHFR was determined to be expressed from the pGL3-TKpr-DHFR-TKpolyA plasmid through western blot analysis with the reagents. Although alternate western blot reagents were optimized for hCrmA expression, there were problems with the DHFR detection. However, in the meantime, RT-PCR analysis confirmed DHFR expression from the pRcAd5-ecl-3-lox plasmid (FIG. 7). In this analysis, 293 cells were transfected with 2ug of the appropriate DHFR expressing plasmid, pMT010/a+, pGL3-TKpr-DHFR-TKpA or pRcAd5-ecl-3-lox or mock transfected. After 48 hours cells were harvested and 1ug of RNA used in a one-step RT-PCR reaction with mDHFR specific primers. Faint bands in lanes 2 & 3 do indicate these primers can also detect hDHFR but is clear all plasmid transfected cells expressed DHFR.

### Analysis of the lox region

While the puromycin cassette functionality was confirmed by transforming E.coli and plating onto agar-puro plates, the Lox region was also analyzed, initially in pRc-Ad5-ecl-2-lox, and then the final pRc-Spacer plasmid. To do so, a Cre recombinase expressing plasmid and a plasmid containing lox sites flanking a gene of interest was needed.

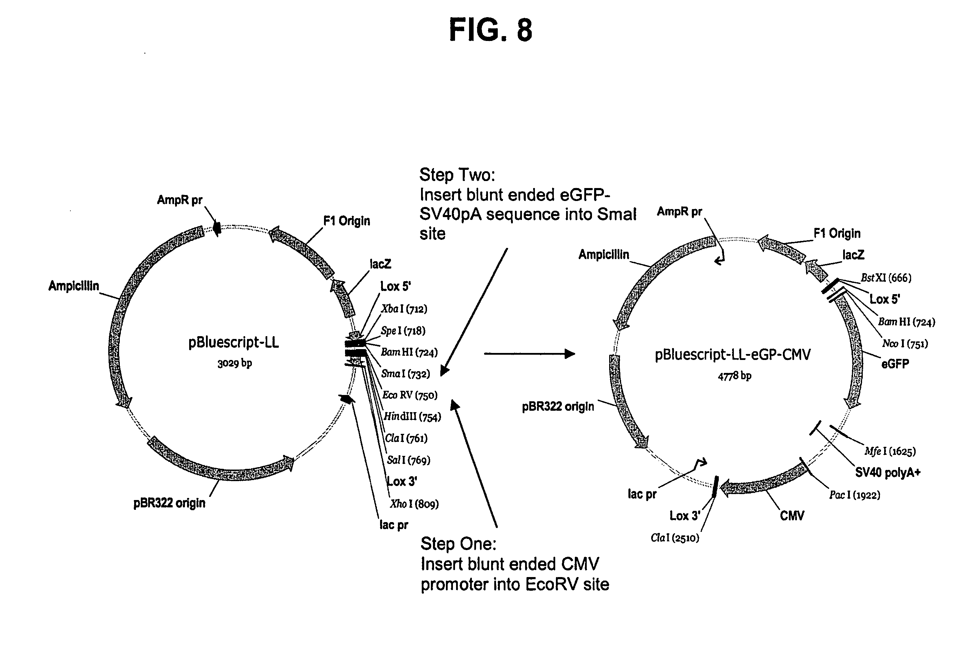

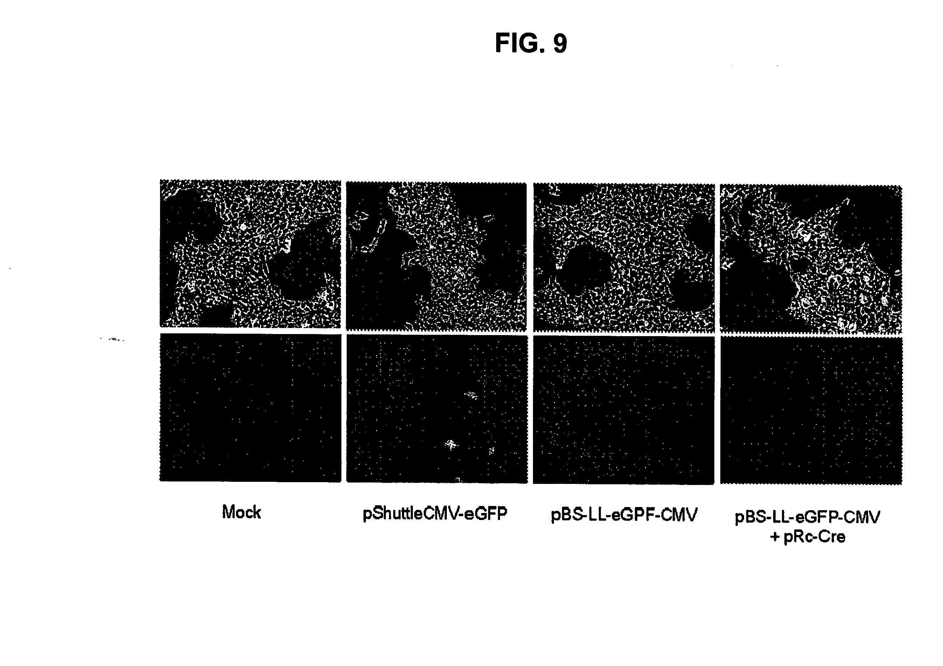

The generation of the Cre recombinase expressing plasmid, pRcCMV-IVS-Cre-puro was completed by inserting a PCR fragment of Cre from the AdCMVCre obtained from GTC. Cre expression and functionality were tested by co-transfecting 293 cells with pRcCMV-IVS-Cre-puro and pBluescript-LL-eGFP-CMV (illustrated in FIG. 8). The construction of pBluescript-LL-eGPF-CMV was completed through blunt end cloning of the CMV promoter into the EcoRV site and eGPF-polyA into the SmaI site of pBluescript-LL. If the Cre recombinase works then the eGFP-CMV sequence will rearrange bringing eGFP under control of CMV and cells are fluorescent. pRcCMV-IVS-Cre-puro works as illustrated in FIG. 9. When cells were transfected with 2ug of pBluescript-LL-eGFP-CMV (shortened name, pBS-LL-eGFP-CMV) there was no fluorescense. However when they were co-transfected with 0.5ug of pRcCMV-IVS-Cre-puro (pRc-Cre shortened name) there were cells fluoresced indicating that pRc-Cre expressed functional Cre recombinase.

With respect to point a plasmid containing lox sites flanking a gene of interest, human growth factor was used as a gene of interest. pBluescript II KS (+) was the backbone plasmid for generating the test vector, pBS-Lox-RSVpr-GH-Lox, and controls, pBS-Lox-OGH-Lox and pBluescript-LL. Furthermore, pBS-Lox-OGH-Lox can be used to compare different promoters for expression of hGH, while pBluescript-LL is flexible to allow different promoter/transgene expression. Southern blotting is used for detecting successful cre/lox mechanics.

### CrmA cassette

A CrmA cassette was previously generated using the pEF-Bos plasmid, which contains the hEF1 promoter and a 3'UTR with GM-CSF polyA. Expression of CrmA was confirmed through western blot analysis using the BD Biosciences anti-CrmA antibody as the probe with the new western blot gels and reagents. The pEF-hCrmA plasmid was altered to contain a 5' AvrII site, and 3' NheI site so that the entire cassette could be excised through AvrII/NheI. This fragment was inserted into NheI linearized pRc-Ad5-ecl-3-lox. After determining it was necessary to switch to another type of electrocompetent bacteria, it was confirmed through enzyme digests and sequencing that pRc-Ad5-ecl-4-lox was generated. In this instance, STBL2 electrocompetent bacteria was used, but STBL4 may be used for future cloning.

### Ad5 sequence 3511-5000

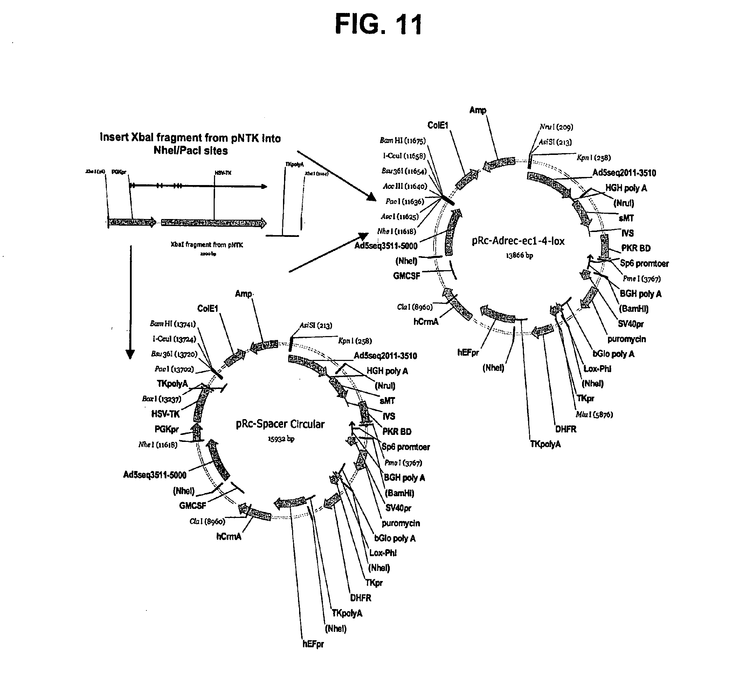

A second piece of the Ad5 sequence, Ad5seq3511-5000, was generated flanked by AvrII and NheI ends, using PCR methods and ligated the fragment into into NheI linearized pRc-Ad5-ecl-4-lox. This plasmid is now known as pRc-Adrec-ecl-4-lox (see FIG. 11) and is ready for insertion of the final cassette.

### PGK-TK cassette

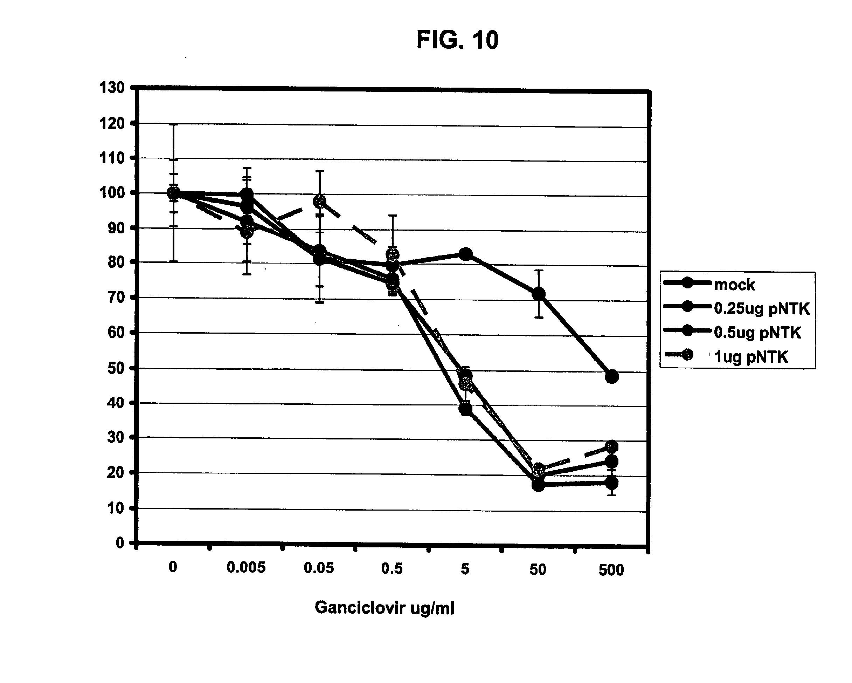

With respect to the final piece, the PGK-TK cassette, a plasmid called pNTK was obtained and TK expression was confirmed in 293 cells transfected with 2ug of pNTK using western blot analysis. The functionality of TK expressed from pNTK was confirmed in a cytoxicity assay (see FIG. 10).

In this analysis 293 cells plated in a 96 well plate were mock transfected or transfected with 0.25ug, 0.5ug or 1ug of pNTK plasmid. After 5 hours, cells were refeed with normal media, and then 16 hours later were treated with ganciclovir at the doses indicated. Cytoxicity was measured at 48 hours following ganciclovir treatment using the WST-1 reagent. As can be seen in figure 5, at the 5ug/ml and 50ug/ml doses, there was a large difference between mock transfected cells and those treated with pNTK plasmid indicating that TK is functional.

To generate the final pRc-Sapcer plasmid (as shown in FIG. 11), the initial plan was to excise the cassette with XbaI from pNTK and in the first instant use adaptor oligos to generate AscI ends on this cassette for cloning into the AscI site in the Lox multi-cloning site. However, due to various difficulties, the cassette was amplified from pNTK and cloned into NheI/PacI in pRc-Adrec-ecl-4-lox. As the plasmid has been sequenced sequentially throughout the cloning period, the sequence of pRc-Spacer is confirmed with a series of digests and PCR on the various cassettes.

### Generation of Test Virus

To analyze the properties of the proposed 293 cell line, a test virus is required. Ad5.eGFP is synthesized by recombining a shuttle vector containing eGFP (enhanced GFP) and pAdEasy vector. The resulting recombinant genome is initially rescued on standard 293 cells, and then the primary lysate stock stored until a comparison can be made on the new cells line versus a standard 293 cell line for RCA breakthrough.

The invention further relates to the following numbered paragraphs:
1. A mammalian cell comprising a non-homologous spacer element inserted into an adenovirus nucleotide sequence, wherein the spacer element represses undesirable homologous recombination.
2. The mammalian cell of paragraph 1 wherein the cell is a human embryonic kidney cell.
3. The mammalian cell of paragraph 1 or 2 wherein the spacer element is inserted by site directed integration at a specific site in the adenovirus nucleotide sequence.
4. The mammalian cell of paragraph 3 wherein the site is after the end of the E1B transcription unit but in front of the pIX transcription unit.
5. The mammalian cell of paragraph 3. wherein the site is after the ITR and packaging sequences but before the E1A transcription start site.
6. The mammalian cell of paragraph 3 wherein the site is after the E1A sequences but before the E1B sequences.
7. The mammalian cell of any one of paragraphs 1 to 6 wherein the spacer element comprises one or more regulatory elements.
8. The mammalian cell of paragraph 7 wherein the one or more regulatory elements are selected from the group consisting of promoters, enhancers, insulators, polyadenylation and termination signals.
9. The mammalian cell of any one of paragraphs 1 to 8 wherein the spacer element is about 2000 to about 3000 base pairs.
10. The mammalian cell of any one of paragraphs 1 to 8 wherein the spacer element is at least about 2000 base pairs.
11. The mammalian cell of any one of paragraphs 1 to 8 wherein the spacer element is at least about 4000 base pairs.
12. The mammalian cell of any one of paragraphs 1 to 8 wherein the spacer element is at least about 6000 base pairs.
13. The mammalian cell of any one of paragraphs 1 to 12 wherein the spacer element comprises one or more integration sequence elements.
14. The mammalian cell of paragraph 13 wherein the one or more integration sequence elements is selected from the group consisting of lox, frt, attB phiC31 integration site sequences.
15. The mammalian cell of any one of paragraphs 1 to 14 wherein the spacer element does not express any genes.
16. The mammalian cell of any one of paragraphs 1 to 14 wherein the spacer element expresses one or more genes advantageous for adenovirus or protein production.
17. The mammalian cell of paragraph 16 wherein the one or more genes is selected from the group consisting of anti-apoptotic genes, growth promoting genes, kinases and selectable markers.
18. The mammalian cell of paragraph 17 wherein the anti-apoptotic genes are selected from the group consisting of CrmA and Bcl-2.
19. The mammalian cell of paragraph 17 wherein the growth promoting genes are selected from the group consisting of dominant negative double-stranded RNA-dependent protein kinase (PKR), adenoviral VA gene, SV40 T-antigen gene and cytokines.
20. The mammalian cell of paragraph 17 wherein the selectable markers are selected from the group consisting of neomycin, puromycin, zeomycin, bleomycin and ABC transporter.
21. The mammalian cell of any one of paragraphs 16 to 20 wherein the expression of one or more genes is driven by a constitutive promoter, an inducible promoter or a regulatable promoter.
22. The mammalian cell of paragraph 21 wherein the constitutive promoter is selected from the group consisting-of CMV, RSV, SV40, PKG and TK.
23. The mammalian cell of paragraph 21 wherein the inducible promoter or regulatable promoter is selected from the group consisting of a metallothionein promoter or a tetR system.
24. The mammalian cell of any one of paragraphs 16 to 23 wherein the one or more genes are inserted into the spacer element randomly.
25. The mammalian cell of any one of paragraphs 16 to 23 wherein the one or more genes are inserted into the spacer element by site directed integration.
26. The mammalian cell of any one of paragraphs 16 to 25 wherein the one or more genes is a dominant negative double-stranded RNA-dependent protein kinase.
27. The mammalian cell of any one of paragraphs 15 to 26 wherein the one or more genes is a mutant adenoviral VA gene.
28. The mammalian cell of paragraph 26 or 27 wherein the expression of a dominant negative double-stranded RNA-dependent protein kinase or mutant adenoviral VA gene is driven by a tetracycline repressor system.
29. The mammalian cell of paragraph 26 or 27 wherein the expression of a dominant negative double-stranded RNA-dependent protein kinase or mutant adenoviral VA gene is driven by a metallothionein promoter.
30. The mammalian cell of paragraph 29 wherein the metallothionein promoter is sheep metallothionein promoter la gene promoter.
31. The mammalian cell of any one of paragraphs 1 to 30 comprising at two or more non-homologous spacer elements inserted into the adenovirus nucleotide sequence.
32. The mammalian cell of paragraph 31 wherein the two or more non-homologous spacer elements are identical.
33. The mammalian cell of paragraph 32 wherein the two or more non-homologous spacer elements are different.
33. A method of repressing homologous recombination events in a mammalian cell comprising inserting a non-homologous spacer element into an adenovirus nucleotide sequence.
34. The method of paragraph 33 comprising the mammalian cells of any one of paragraphs 1 to 32.
35. A mammalian cell comprising a non-homologous spacer element inserted into an adenovirus nucleotide sequence, wherein the spacer element reduces the presence of replication-competent adenovirus (RCA) in adenovirus vector production.
36. The mammalian cell of paragraph 35 wherein the frequency of RCA generated in adenovirus vector production is reduced by at least ten fold compared to production in the parental mammalian cell without a non-homologous spacer element.
37. The mammalian cell of paragraph 35 wherein the frequency of RCA generated in adenovirus vector production is reduced by at least hundred fold compared to production in the parental mammalian cell without a non-homologous spacer element.
38. The mammalian cell of paragraph 35 wherein the cell is a human embryonic kidney cell.
39. The mammalian cell of any of the paragraph 35 to 38 wherein the spacer element is inserted by site directed integration at a specific site in the adenovirus nucleotide sequence.
40. The mammalian cell of paragraph 39 wherein the site is after the end of the E1B transcription unit but in front of the pIX transcription unit.
41. The mammalian cell of paragraph 39 wherein the site is after the ITR and packaging sequences but before the E1A transcription start site.
42. The mammalian cell of paragraph 39 wherein the site is after the E1A sequences but before the E1B sequences.
43. The mammalian cell of any one of paragraphs 35 to 42 wherein the spacer element comprises one or more regulatory elements.
44. The mammalian cell of paragraph 43 wherein the one or more regulatory elements are selected from the group consisting of promoters, enhancers, insulators, polyadenylation and termination signals.
45. The mammalian cell of any one of paragraphs 35 to 44 wherein the spacer element is about 2000 to about 3000 base pairs.
46. The mammalian cell of any one of paragraphs 35 to 44 wherein the spacer element is at least about 2000 base pairs.
47. The mammalian cell of any one of paragraphs 35 to 44 wherein the spacer element is at least about 4000 base pairs.
48. The mammalian cell of any one of paragraphs 35 to 44 wherein the spacer element is at least about 6000 base pairs.
49. The mammalian cell of any one of paragraphs 35 to 48 wherein the spacer element comprises one or more integration sequence elements.
50. The mammalian cell of paragraph 49 wherein the one or more integration sequence elements is selected from the group consisting of lox, frt, attB phiC31 integration site sequences.
51. The mammalian cell of any one of paragraphs 35 to 50 wherein the spacer element does not express any genes.
52. The mammalian cell of any one of paragraphs 35 to 50 wherein the spacer element expresses one or more genes advantageous for adenovirus or protein production.
53. The mammalian cell of paragraph 52 wherein the one or more genes is selected from the group consisting of anti-apoptotic genes, growth promoting genes, kinases and selectable markers.
54. The mammalian cell of paragraph 53 wherein the anti-apoptotic genes are selected from the group consisting of CrmA and Bcl-2.
55. The mammalian cell of paragraph 52 wherein the growth promoting genes are selected from the group consisting of dominant negative double-stranded RNA-dependent protein kinase (PKR), adenoviral VA gene, SV40 T-antigen gene and cytokines.
56. The mammalian cell of paragraph 52 wherein the selectable markers are selected from the group consisting of neomycin, puromycin, zeomycin, bleomycin and ABC transporter.
57. The mammalian cell of any one of paragraphs 52 to 56 wherein the expression of one or more genes is driven by a constitutive promoter, an inducible promoter or a regulatable promoter.
58. The mammalian cell of paragraph 57 wherein the constitutive promoter is selected from the group consisting of CMV, RSV, SV40, PKG and TK.
59. The mammalian cell of paragraph 57 wherein the inducible promoter or regulatable promoter is selected from the group consisting of a metallothionein promoter or a tetR system.
60. The mammalian cell of any one of paragraphs 52 to 59 wherein the one or more genes are inserted into the spacer element randomly.
61. The mammalian cell of any one of paragraphs 52 to 59 wherein the one or more genes are inserted into the spacer element by site directed integration.
62. The mammalian cell of any one of paragraphs 52 to 61 wherein the one or more genes is a dominant negative double-stranded RNA-dependent protein kinase.
63. The mammalian cell of any one of paragraphs 51 to 62 wherein the one or more genes is a mutant adenoviral VA gene.
64. The mammalian cell of paragraph 62 or 63 wherein the expression of a dominant negative double-stranded RNA-dependent protein kinase or mutant adenoviral VA gene is driven by a tetracycline repressor system.
65. The mammalian cell of paragraph 62 or 63 wherein the expression of a dominant negative double-stranded RNA-dependent protein kinase or mutant adenoviral VA gene is driven by a metallothionein promoter.
66. The mammalian cell of paragraph 65 wherein the metallothionein promoter is sheep metallothionein promoter la gene promoter.
67. The mammalian cell of any one of paragraphs 35 to 66 comprising at two or more non-homologous spacer elements inserted into the adenovirus nucleotide sequence.
68. The mammalian cell of paragraph 67 wherein the two or more non-homologous spacer elements are identical.
69. The mammalian cell of paragraph 68 wherein the two or more non-homologous spacer elements are different.
70. The mammalian cell of any one of paragraphs 35 to 69 wherein is the reduced frequency is due to inefficient packaging and propagation.
71. The mammalian cell of paragraph 70 wherein the inefficient packaging and propation is due to the resulting size of the recombinant non-homologous spacer element inserted into the adenovirus nucleotide sequence.
72. A method of reducing the frequency of generation of RCA in a mammalian cell comprising inserting a non-homologous spacer element into an adenovirus nucleotide sequence.
73. The method of paragraph 72 comprising the mammalian cells of any one of paragraphs 35 to 71.

## Claims

1. A mammalian packaging or production cell based on a HEK 293 cell, said cell being capable of complementing growth of adenovirus vectors deficient in the E1A and E1B genes, said mammalian packaging or production cell comprising a non-homologous spacer element of at least 2000 base pairs inserted into a specific site in the adenovirus nucleotide sequence already existing in HEK 293 cells wherein said non-homologous spacer element
- does not interfere with the function of E1A and E1B genes, and
- is non homologous with any adenoviral nucleotide sequence;
said cell having a reduced probability of undesirable replication-competent adenovirus (RCA) in adenovirus vector production if compared with a HEK 293 cell lacking said non-homologous spacer element.

2. The mammalian cell of claim 1 wherein said spacer element is inserted by site directed integration at a site located after the end of the E1B transcription unit but in front of the pIX transcription unit into the adenovirus nucleotide sequence already existing in HEK 293 cells.

3. The mammalian cell of any one of claims 1 to 2 wherein the spacer element comprises one or more regulatory elements selected from the group consisting of promoters, enhancers, insulators, polyadenylation and termination signals.

4. The mammalian cell of any one of claims 1 to 3 wherein the spacer element is about 2000 to about 3000 base pairs.

5. The mammalian cell of any one of claims 1 to 4 wherein the spacer element is at least about 4000 base pairs, or is at least about 6000 base pairs.

6. The mammalian cell of any one of claims 1 to 5 wherein the spacer element comprises one or more integration sequence elements, preferably said one or more integration sequence elements being selected from the group consisting of lox, frt, attB and phiC31 integration site sequences.

7. The mammalian cell of any one of claims 1 to 6 wherein the spacer element does not express any genes.

8. The mammalian cell of any one of paragraphs 1 to 6 wherein the spacer element expresses one or more genes advantageous for adenovirus or protein production.

9. The mammalian cell of claim 8 wherein the one or more genes is selected from the group consisting of anti-apoptotic genes, growth promoting genes, kinases and selectable markers.

10. The mammalian cell of claim 9 wherein the genes are selected from the group consisting of CrmA, Bcl-2, dominant negative double-stranded RNA-dependent protein kinase (PKR), adenoviral VA gene, SV40 T-antigen gene and cytokines and/or wherein the selectable markers are selected from the group consisting of neomycin, puromycin, zeomycin, bleomycin and ABC transporter.

11. The mammalian cell of any one of claims 1 to 10 comprising two or more non-homologous spacer elements inserted into the preexisting adenovirus nucleotide sequence.

12. The mammalian cell of claim 11 wherein
- said spacer element is inserted by site directed integration at a site located after the end of the E1B transcription unit but in front of the pIX transcription unit into the adenovirus nucleotide sequence already existing in HEK 293 cells, and
- a further spacer element is inserted after the ITR and packaging sequences but before the E1A transcription start site and/or after the E1A sequences but before the E1B sequences.

13. The mammalian cell of claim 1-12 wherein the frequency of replication-competent adenovirus (RCA) generated in the introduced adenovirus vector production is reduced by at least ten fold compared to production in a HEK 293 cell without said non-homologous spacer element.

14. The mammalian cell of any one of claim 1 to 13 wherein
the reduced frequency of RCA is due to inefficient packaging and propagation of the adenovirus vector and/or
the reduced frequency of RCA is due to repression of undesirable homologous recombination between the adenovirus sequence already existing in the HEK 293 cell and a newly introduced adenovirus vector sequence.

15. A method of repressing homologous recombination events between the already existing adenovirus sequence in the HEK 293 mammalian cell and a newly introduced adenovirus vector sequence comprising inserting a non-homologous spacer element as defined in any of claims 1 to 14 into the already existing adenovirus nucleotide sequence.

16. Use of a cell according to any of claims 1 to 14 in a method for adenovirus vector production wherein the production of replication-competent adenovirus (RCA) is undesired.

## Patentansprüche

1. Eine Säugetier-Verpackungs- oder Produktionszelle, basierend auf einer HEK 293 Zelle, wobei die Zelle das Wachstum von Adenovirus-Vektoren, die in den E1A- und E1B-Genen fehlerhaft sind, komplementieren kann, wobei die Säugetier-Verpackungs- oder Produktionszelle ein nicht-homologes Spacer-Element aus mindestens 2000 Basenpaaren, das in eine spezifische Stelle in der Adenovirus-Nukleotidsequenz, die bereits in HEK 293 Zellen existiert, eingefügt wird, umfasst, wobei das nicht-homologe Spacer-Element
- nicht mit der Funktion von E1A- und E1B-Genen interferiert, und
- nicht-homolog ist mit irgendeiner adenoviralen Nukleotid-Sequenz;
wobei die Zelle eine verringerte Wahrscheinlichkeit von unerwünschtem Replikations-kompetenten Adenovirus (RCA) in der Adenovirus-Vektorproduktion hat, im Vergleich zu einer HEK 293 Zell, der das nicht-homologe Spacer-Element fehlt.

2. Die Säugetierzelle nach Anspruch 1, wobei das Spacer-Element durch Orts-gerichtete Integration an einer Stelle, die sich nach dem Ende der E1B-Transkriptionseinheit aber vor der pIX-Transkriptionseinheit befindet, in die Adenovirus-Nukleotidsequenz, die bereits in HEK 293 Zellen existiert, eingefügt wird.

3. Die Säugetierzelle nach einem der Ansprüche 1 bis 2, wobei das Spacer-Element ein oder mehrere regulatorische Elemente umfasst, die aus der Gruppe, die aus Promotern, Verstärkern, Isolatoren, Polyadenylierung und Abbruchssignalen besteht, ausgewählt wird.

4. Die Säugetierzelle nach einem der Ansprüche 1 bis 3, wobei das Spacer-Element ungefähr 2000 bis ungefähr 3000 Basenpaare hat.

5. Die Säugetierzelle nach einem der Ansprüche 1 bis 4, wobei das Spacer-Element mindestens 4000 Basenpaare hat oder mindestens 6000 Basenpaare hat.

6. Die Säugetierzelle nach einem der Ansprüche 1 bis 5, wobei das Spacer-Element ein oder mehrere Integrations-Sequenzelemente umfasst, wobei das eine oder die mehreren Integrations-Sequenzelemente bevorzugt aus der Gruppe, die aus lox, frt, attB und phiC31 Integrationsstellensequenzen besteht, ausgewählt wird.

7. Die Säugetierzelle nach einem der Ansprüche 1 bis 6, wobei das Spacer-Element keine Gene exprimiert.

8. Die Säugetierzelle nach einem der Ansprüche 1 bis 6, wobei das Spacer-Element ein oder mehrere Gene exprimiert, die für die Adenovirus- oder Proteinproduktion vorteilhaft sind.

9. Die Säugetierzelle nach Anspruch 8, wobei das eine oder die mehreren Gene aus der Gruppe, die aus antiapoptotischen Genen, wachstumsfördernden Genen, Kinasen und auswählbaren Markern besteht, ausgewählt werden.

10. Die Säugetierzelle nach Anspruch 9, wobei die Gene aus der Gruppe, die aus CrmA, BcI-2, dominant negative Doppelstrang RNA-abhängige Proteinkinase (PKR), adenovirales VA Gen, SV40 T-Antigengen und Cytokinen besteht, ausgewählt werden und/oder wobei die auswählbaren Markern aus der Gruppe, die aus Neomycin, Puromycin, Zeomycin, Bleomycin und ABC Transporter besteht, ausgewählt werden.

11. Die Säugetierzelle nach einem der Ansprüche 1 bis 10, die zwei oder mehrere nicht-homologe Spacer-Elemente, die in die vorher vorhandene Adenovirus-Nukleotidsequenz eingefügt werden, umfasst.

12. Die Säugetierzelle nach Anspruch 11, wobei
- das Spacer-Element durch Orts-gerichtete Integration an eine Stelle, die sich nach dem Ende der E1B-Transkriptionseinheit aber vor der pIX-Transkriptionseinheit befindet, in die Adenovirus-Nukleotidsequenz, die bereits in HEK 293 Zellen existiert, eingefügt wird, und
- ein weiteres Spacer-Element nach dem ITR und den Verpackungs-Sequenzen aber vor der E1A-Transkriptions-Startstelle und/oder nach den E1A-Sequenzen aber vor den E1B-Sequenzen eingefügt wird.

13. Die Säugetierzelle nach Anspruch 1-12, wobei die Frequenz des Replikations-kompetenten Adenovirus (RCA), die in der eingeführten Adenovirus-Vektorproduktion erzeugt wird, um mindestens das 10-fache verglichen mit der Produktion in einer HEK 293 Zelle ohne das nicht-homologe Spacer-Element verringert wird.

14. Die Säugetierzelle nach einem der Ansprüche 1 bis 13, wobei
- die verringerte Frequenz von RCA auf das ineffiziente Verpacken und die Propagation des Adenovirus-Vektors zurückzuführen ist, und/oder
- die verringerte Frequenz von RCA auf die Repression der unerwünschten homologen Rekombination zwischen der Adenovirussequenz, die bereits in der HEK 293 Zelle existiert, und einer neu eingeführten Adenovirus-Vektorsequenz zurückzuführen ist.

15. Ein Verfahren des Unterdrückens homologer rekombinanter Ereignisse zwischen der bereits existierenden AdenovirusSequenz in der HEK 293 Säugetier-Zelle und einer neu eingeführten Adenovirus-Vektorsequenz, das das Einführen eines nicht-homologen Spacer-Elements, wie in einem der Ansprüche 1 bis 14 definiert, in die bereits existierende Adenovirus-Nukleotidsequenz umfasst.

16. Verwendung einer Zelle gemäß einem der Ansprüche 1 bis 14 in einem Verfahren zur Adenovirus-Vektorproduktion, wobei die Produktion des Replikations-kompetenten Adenovirus (RCA) unerwünscht ist.

## Revendications

1. Cellule d'encapsidation ou de production issue de mammifère, à base de cellule HEK 293, ladite cellule pouvant complémenter la croissance de vecteurs adénoviraux déficients en gènes E1A et E1B, ladite cellule d'encapsidation ou de production issue de mammifère comprenant un élément d'espacement non homologue d'au moins 2 000 paires de bases inséré dans un site spécifique dans la séquence nucléotidique adénovirale déjà existante dans les cellules HEK 293, ledit élément d'espacement non homologue
- n'interférant pas avec la fonction des gènes E1A et E1B, et
- n'étant pas homologue à une séquence nucléotidique adénovirale ;
ladite cellule ayant une probabilité réduite de présenter un adénovirus compétent pour la réplication indésirable (RCA) dans la production de vecteur adénoviral par rapport à une cellule HEK 293 dépourvue dudit élément d'espacement non homologue.

2. Cellule de mammifère selon la revendication 1, dans laquelle ledit élément d'espacement est inséré par intégration dirigée sur un site situé après l'extrémité de l'unité de transcription de E1B mais avant l'unité de transcription de pIX dans la séquence nucléotidique adénovirale déjà existante dans les cellules HEK 293.

3. Cellule de mammifère selon l'une quelconque des revendications 1 à 2, dans laquelle l'élément d'espacement comprend un ou plusieurs éléments régulateurs choisis dans le groupe comprenant les promoteurs, les amplificateurs, les isolants, les signaux de polyadénylation et de terminaison.

4. Cellule de mammifère selon l'une quelconque des revendications 1 à 3, dans laquelle l'élément d'espacement a environ 2 000 à environ 3 000 paires de bases.

5. Cellule de mammifère selon l'une quelconque des revendications 1 à 4, dans laquelle l'élément d'espacement a au moins environ 4 000 paires de base ou a au moins environ 6 000 paires de bases.

6. Cellule de mammifère selon l'une quelconque des revendications 1 à 5, dans laquelle l'élément d'espacement comprend un ou plusieurs éléments de séquence d'intégration, de préférence lesdits un ou plusieurs éléments de séquence d'intégration étant choisis dans le groupe comprenant les séquences de site d'intégration lox, frt, attB et phiC31.

7. Cellule de mammifère selon l'une quelconque des revendications 1 à 6, dans laquelle l'élément d'espacement n'exprime pas de gènes.

8. Cellule de mammifère selon l'un quelconque des paragraphes 1 à 6, dans laquelle l'élément d'espacement exprime un ou plusieurs gènes avantageux pour la production d'adénovirus ou de protéine.

9. Cellule de mammifère selon la revendication 8, dans laquelle lesdits uns ou plusieurs gènes sont choisis dans le groupe consistant en gènes antiapoptotiques, en gènes promoteurs de croissance, kinases et marqueurs sélectionnables.

10. Cellule de mammifère selon la revendication 9, dans laquelle les gènes sont choisis dans le groupe comprenant CrmA, Bcl-2, une protéine kinase dépendante d'un ARN à double brin négatif dominant (PKR), un gène VA adénoviral, un gène d'antigène T SV40 et des cytokines et/ou dans laquelle les marqueurs sélectionnables sont choisis dans le groupe comprenant la néomycine, la puromycine, la zéomycine, la bléomycine et un transporteur ABC.

11. Cellule de mammifère selon l'une quelconque des revendications 1 à 10, comprenant deux éléments d'espacements non homologues ou plus, insérés dans la séquence nucléotidique adénovirale préexistante.

12. Cellule de mammifère selon la revendication 11, dans laquelle
- ledit élément d'espacement est inséré par intégration dirigée sur un site situé après la fin de l'unité de transcription de E1B mais avant l'unité de transcription de pIX dans la séquence nucléotidique adénovirale déjà existante dans des cellules HEK 293, et
- un autre élément d'espacement est inséré après l'ITR et les séquences d'encapsidation mais avant le site de début de transcription de E1A et/ou après les séquences de E1A mais avant les séquences de E1B.

13. Cellule de mammifère selon la revendication 1 à 12, dans laquelle la fréquence d'adénovirus compétent pour la réplication (RCA) générée dans la production de vecteur adénoviral introduit est réduite d'au moins facteur dix par rapport à la production dans une cellule HEK 293 sans ledit élément d'espacement non homologue.

14. Cellule de mammifère selon l'une quelconque des revendications 1 à 13, dans laquelle
la fréquence réduite de RCA est due à une encapsidation et une propagation inefficace du vecteur adénoviral et/ou
la fréquence réduite de RCA est due à la répression de la recombinaison homologue indésirable entre la séquence adénovirale déjà existante dans la cellule HEK 293 et une séquence de vecteur adénoviral nouvellement introduite.

15. Procédé de répression d'événements de recombinaison homologue entre la séquence adénovirale déjà existante dans la cellule de mammifère HEK 293 et une séquence de vecteur adénoviral nouvellement introduite comprenant l'insertion d'un élément d'espacement non homologue tel que défini dans l'une quelconque des revendications 1 à 14 dans la séquence nucléotidique adénovirale existante.

16. Utilisation d'une cellule selon l'une quelconque des revendications 1 à 14, dans un procédé de production de vecteur adénoviral, dans laquelle la production de l'adénovirus compétent pour la réplication (RCA) est indésirable.
